# EUROPEAN PATENT APPLICATION

(11) **EP 2 193 787 A1**
(43) Date of publication of application: **09.06.2010**
(21) Application number: 08425771.6
(22) Date of filing: 02.12.2008
(51) Int. Cl.: A61K 9/20

(54) **Formulations for systemic buccal delivery comprising s-adenosylmethionine, their preparation and use**

(71) Applicant: Stramentinoli, Giorgio, 20141 Milano (IT); Busetti, Cesare, 20149 Milano (IT)
(72) Inventor: Stramentinoli, Giorgio, 20141 Milano (IT); Busetti, Cesare, 20149 Milano (IT)
(74) Representative: Gervasi, Gemma

(57) **Abstract**

Formulations for systemic buccal delivery, in particular chewing gums, chewable tablets, orodispersible tablets, oromucosal preparations comprising sulpho-adenosyl-L-methionine (SAMe) which allows the absorption of the active principle through the oral mucosa are described.

## Description

### Field of the invention

The present invention relates to oral compositions based on S-adenosylmethionine, or salts thereof, particularly designed for systemic buccal delivery.

### State of the art

As it is known sulpho-adenosyl-L-methionine (SAMe) is a well known biological donor of methyl groups.

On the other hand it is also known that for a correct and efficient therapy the appropriate way of administration and adsorption of an active principle play an essential role

Among the various ways of administration of the above said active principle the parenteral one is the one permitting a quick and efficient treatment but unfortunately this way has a very low compliance especially for long time therapies, it is rather expensive and practically does not allow self-medication.

In view of the above said problems when a quick effect is not requested the active principle is normally administered per os (both in liquid or solid form) for example by capsules, tablets etc.

However the oral administration of SAMe suffers the degradation processes of the active principle in the liver (hepatic first-pass) which causes a low bio-availability, moreover the enzymes of the digestive tract contribute also to reduce the bio-availability of the compound.

Moreover it should also be considered that some oral form can be difficult to swallow especially for old people or little children in particular when a high dosage in active principle is requested.

In particular, in EP 136 464 it is reported that while SAMe salts are practically unabsorbed when administered orally they are adsorbed to a considerable extent if administered directly in the intestine and gastro-resistant forms are described in order to overcome the problem.

However also these forms have their drawbacks since the active principle must be maintained at a rather low (acid) pH and the adsorption time is rather long.

US 2007/0160660 describes formulations for oral use comprising SAMe and a high quantity of inositole in order to make the product more stable in respect of temperature and humidity (which are two adverse factors) but such high quantity implies rather large dimension of the tablet and therefore the above said swallowing difficulties.

US 6,759,359 deals with the same problem as the above said EP 136 464 and suggests gastro-resistant soft-gelatin capsules in order to allow a duodenal adsorption, also in this case the same drawbacks (long time of adsorption) are present.

It is evident in view of the above said the importance to make available new formulation capable of overcoming the above said problems.

### Summary of the invention

Formulations for systemic buccal delivery comprising as active principle sulpho-adenosyl-L-methionine are described, in particular in the form of chewing gums, chewable tablets, orodispersible tablets and oromucosal preparations capable of allowing the absorption of said active principle through the oral mucosa.

### Detailed description of the invention

It was now surprisingly found that sulpho-adenosyl-L-methionine (SAMe) can be safely and advantageously administered in a form that allows adsorption through the mouth mucosa as for example chewing gums, chewable tablets, orodispersible tablets, oromucosal preparations.

According to the invention for chewing gums, chewable tablets, oral dispersible tablets and oromucosal preparations it is intended the formulations normally used for analogous purposes.

Medicated chewing gums are described in Eur. Ph. 6.0 as "single-dose preparations with a base consisting mainly of gum that are intended to be chewed but not swallowed". European Pharmacopoeia also states that chewing gums contain one or more active substances which are released by chewing. After dissolution or dispersion of the active substances in saliva, chewing gums are intended to be used for systemic delivery after absorption through the buccal mucosa or from the gastrointestinal tract.

Moreover Eur. Ph. 6.0 describes a dissolution test for medicated chewing gums (2.9.25, page 304) useful to check the dissolution of active substances *in-vitro.*

Generally, the composition of medicated chewing gums includes, apart from active(s) ingredient, gum base, bulk sweeteners, flavours, softeners, etc.

The main components of the gum base are elastomers (polyisobutylene, butyl rubber, etc.), plasticizers (resins, polyvinyl acetate, etc.), texturizers (fats, microwaxes, lipophilic emulsifiers, etc.), and fillers (calcium carbonate, talc, etc.).

Various manufacturing methods can be used to prepare medicated chewing gums. The first one is the "conventional melting method" that involves various steps, briefly: melting of the gum base, addition and blending of the other ingredients, kneading and rolling of the mixture, cutting and seasoning of the gums. If necessary the process can include a final coating.

The "cooling and grinding method" requires the refrigeration of the gum composition until it becomes sufficiently brittle to make possible the grinding. This method requires special equipment and a careful control of the humidity during the process and, moreover, can be potentially hazardous if liquid nitrogen is used as cooling agent.

In a preferred embodiment the manufacturing method uses the directly compressible gum bases commonly available on the market (direct compression method) for example:
"Pharmagum^{®}", developed by SPI Pharma, "All In Gum" by Cafosa, or "MedGumBase^{™}" by Gum Base Co just to quote some.

The manufacturing process is faster using the above said method and this allows to overcome the drawbacks of the other methods.

This method is particularly suitable in the case of SAMe since the process involves rather mild conditions and avoids humidity, in fact gum bases for direct compression have very low humidity and normally must be processed in a de-humidified environment.

The gummy matrix of chewing gums assures a good protection against the humidity as shown in Table 1 below, and permits to maintain the KF value (moisture determined in according to Karl Fisher method) well below the 2% limit, that those skilled in the art known to be the maximum allowed to have a good stability of the product.

Moreover the formulations according to the invention allow a protection of the active principle against light, and oxygen effects.

If desired it is easily possible to produce tablets with a double or a multiple layer using suitable machinery.. This techniques can be advantageously used for producing chewing gums containing SAMe and other active principles, such as vitamins, mineral salts, oligoelements, aminoacids, and mixtures thereof, when it is necessary to maintain separate the components or to improve the marketing appealing.

If preferred chewing gums can be coated with a coating layer that has several functions. The first one is to give an immediate and strong taste sensation when the patient starts chewing. The coating can also protect the gum base, and other ingredients in the core, from the oxidation and makes possible to prolong the shelf-life. Chewing gums can be coated with a sugar-coating, or with a sugarless coating, or with a film coating.

Moreover, the coating layer not only acts as a taste enhancer but can also contain active ingredients. In a preferred embodiment active ingredients contained in the coating layer are vitamins, mineral salts, oligoelements, aminoacids, and mixtures thereof of the kind normally used for this kind of formulations.

The coating layer consists mainly of sweeteners and flavours, possible sweeteners are sugarless sweeteners as xylitol, sorbitol, maltitol, isomalt, and the like. Often the low sweet sensation due to the sugarless sweeteners is increased using a high-intensity sweeteners as acesulfame K, acesulfame salts, aspartame, cyclamate and its salts, saccharin and its salts, glycyrrhizin, thaumatin, and the like. The coating layer can also contains other ingredients such as dispersing agents, as well as colouring agents, film formers, and binding agents.

The coating layer may be applied in a coating pan or in a fluid bed equipment. In a preferred embodiment the coating layer is applied by double-press coating, also known as compression-coating. This technique does not require water or other solvents during the application making it more advantageous in comparison with the film coating or the sugar coating procedure above all when hygroscopic active ingredients are used. The manufacturing process is carried out with special rotary tabletting machines that automatically perform following steps: load of the bottom layer of the coating into the die from the hopper, centering of the core on the bottom bed of coating, deposition of the top layer of the coating, compression of the whole system by passing the punches between the compression rolls.

Chewable tablets are solid, single dose preparations intended to be sucked to obtain a local or systemic effect. They are prepared by compression and a dissolution test is prescribed in order to demonstrate the appropriate release of the active substance if the tablets are intended for a systemic effect. Any standard excipient able to provide suitable compression can be used in the chewable tablets, such as mannitol, sorbitol, lactose, sucrose, starches and derivatives, cellulose and derivatives, microcrystalline cellulose, and the like. In a preferred embodiment directly compressible excipients are used, such as, but not limited to, Emdex^{®} (Dextrates), Sugartab^{®} (Compressible sucrose), both available from JRS Pharma, Parteck^{®} M (Mannitol), from type M 100 to type M 300, and Parteck^{®} SI (Sorbitol), from type SI 150 to type SI 450, both available from Merck Chemicals, Maltisorb^{®} (Maltitol), Xylisorb^{®} (Xylitol), both available from Roquette, StarLac^{®} (Lactose-starch compound), available from Meggle, Advantose^{™} FS 95 (Fructose), available from SPI Pharma, Glucidex^{®} IT (maltodextrins and dried glucose syrup), available from Roquette, Avicel^{®} CE-15 (microcrystalline cellulose and guar gum), available from FMC, and the like in the quantities normally used for products of this kind.

The orodispersible tablets, also known as orally dissolving-dispersible tablets (ODT) or fast melt tablets, are "uncoated tablets intended to be placed in the mouth where they disperse rapidly before being swallowed", as described in Eur. Ph. 6.0.

The orodispersable tablets of the present invention can be manufactured with any standard excipient such as diluents, glidants, dry binders, disintegrants, sweeteners, flavours, colors, lubricants, etc.

In a preferred embodiment directly compressible excipients able to produce tablets that disintegrates within a matter of seconds are used, such as, but not limited to, F-Melt^{®}, available from Fuji Chemical Industry Co., Ludiflash^{®}, Ludipress^{®}, both available from BASF, StarLac^{®}, available from Meggle, Avicel^{®} HFE-102, available from FMC, Ceolus^{™} KG-802, available from Asahi Kasei Chem.Co, and the like.

Oromucosal preparations are designed to administer one or more active substances to the oral cavity to obtain a local or a systemic effect.

The European Pharmacopoeia distinguishes several categories of oromucosal preparations; some of them can be particularly useful to reach a systemic effect. So, for example, subingual formulations (sublingual tablets and buccal tablets) are solid, single dose preparations to be applied under the tongue or to the buccal cavity to obtain a systemic effect. Any suitable excipient can be used in accordance with the present invention.

In any embodiments of the present invention the composition can be completed with acceptable excipients, such as diluents, binders, lubricants, glidants, disintegrants, surfactants, colorants, and mixtures thereof.

The particular excipient used depends on the means and the purpose for which the active ingredient is applied.

Preferred diluents are mannitol, sorbitol, xylitol, microcrystalline cellulose, silicified microcrystalline cellulose (e.g., Prosolv^{®} available from JRS Pharma) cellulosic polymers, such as hydroxypropylmethylcellulose and hydroxypropylcellulose. Examples of glidants include, but are not limited to, silicon dioxide, colloidal silicon dioxide, calcium silicate, magnesium silicate, talc, and mixtures thereof.

In any of the embodiments, a binder can be added. Suitable binders include, but are not limited to, methylcellulose, hydroxypropylmethylcellulose, hydroxypropylcellulose, polyvinylpirrolidone, gelatin, polyethylene glycols, starch and starch derivatives, sugars, glucose, sorbitol, natural and synthetic gums (e.g., acacia, alginates, carrageenan, xanthan gum, arabic gum), waxes, combinations thereof, and any other binder substances known to those of skill in the art.

Furthermore, in any of the embodiments, a lubricant can be used. Suitable lubricants include, but are not limited to, calcium stearate, magnesium stearate, glyceryl monostearate, glyceryl behenate, glyceryl palmitostearate, hydrogenated vegetable oil, mineral oil, sodium stearyl fumarate, stearic acid, combinations thereof.

Additionally, in any of the embodiments, disintegrants can also be added to the composition to break up the dosage form. suitable disintegrants include, but are not limited to, starch and starch derivatives, sodium starch glycolate, croscarmellose sodium, crospovidone, alginic acid, microcrystalline cellulose, lower-substituted hydroxypropylcellulose, sodium alginate, and combinations thereof.

If preferred the formulations can contain a sweetener or a high-intensity sweetener to impart a pleasant flavor to the composition. Suitable sweeteners for use in the present invention include natural sweeteners such as sucrose, dextrose, fructose, invert sugar, mannitol, sorbitol, thaumatin, and the like, as well as synthetic sweeteners such as saccharin, aspartame, acesulfame K, cyclamates, sucralose, and other commercial artificial sweeteners well-known to those of skill in the art.

The sweetener is added in the amount sufficient to achieve a desired sweetness. Typically, the sweetener is present in an amount from about 0,01% to about 5%.

The advantages obtained with the formulations according to the invention are evident since all of them allow an easy and pleasant administration, do not require water to swallow and therefor can be taken anywhere, do not present problems of swallowing and are suitable both for acute and prolonged treatment.

Since the active principles dissolves and is adsorbed through saliva its passage in the blood is very quick (few minutes) avoiding the first-pass metabolism and increasing the bio-availability and permitting lower dosage.

According to the invention the quantity of active principle is normally comprised between 50 and 1000 mg, preferably is comprised between 100 and 800 mg, and more preferably is comprised between 200 and 400 mg.

If desired the formulations according to the invention can contain also vitamins, aminoacids, mineral salts or oligoelements. In any embodiments, nutraceutically active agents can include, for example, dietary supplements, minerals, vitamins, and the like, and combinations thereof. Exemplary nutraceutically active agents include, e.g., vitamin A, vitamin D, vitamin E, vitamin B1 and derivatives thereof, vitamin B2 and derivatives thereof, vitamin B6 and derivatives thereof, vitamin C and derivatives thereof, vitamin B12 and derivatives thereof, folic acid and folates, acetyl-L-carnitine, Co-enzyme Q10, calcium, magnesium, iron, selenium, chromium, zinc, proteins, amino acids, alpha-lipoic acid, silymarin, oligosaccharides, and the like, and mixuters thereof.

Moreover, in order to minimise the unpleasant taste due to the SAMe acidity, pH-modifying agents can be added; said pH modifying agents are for example chosen among amino acids (as for examples L-Lysine or L-arginine), carbonates or a bicarbonate, a phosphate or a citrate salt of the kind normally used for oral formulations.

The chewing gums, the chewable tablets, the orodispersible tablets, and the oromucosal preparations according to the invention can be easily prepared according to the techniques and methods known in the art for this kind of products.

For example, the SAMe, as well as the other active ingredients if present, is mixed with the directly compressible excipients together with the other appropriate excipients (e.g., binders, glidants, disintegrants, lubricants, sweeteners, flavours, colourants, etc.) for a sufficient time to reach the uniformity of distribution. Then the mixture is loaded to a tabletting machine and compressed following standard procedures.

If the use of granulation becomes necessary to improve the properties of the mixture, the preferred method is the dry granulation that can be performed both in a tabletting machine and in a roller compactor.

The invention will be better illustrated in the light of the following examples.

### Example 1 (Chewing Gums)

| | | | |
|---|---|---|---|
| SAMe Tosylate | | mg | 384,2 |
| All-In-Gum SF | (Cafosa) | mg | 2114,8 |
| L-Arginine | (Kyowa) | mg | 192,0 |
| Mint flavour powder | (Firmenich) | mg | 25,0 |
| Mint flavour liquid | (Firmenich) | mg | 15,0 |
| Magnesium stearate | | mg | 36,0 |
| Aspartame | (Ajinomoto) | mg | 5,0 |
| Sipernat 50S | (Evonik Degussa) | mg | 28,0 |
| Talin | (Overseal) | mg | 0,01 |

The gum base (All-In-Gum SF) is loaded in a suitable blender and the lumps, if present, are broken. In the same blender, SAMe tosylate, L-arginine, aspartame, mint flavour powder, and Talin are incorporated. Following, the mint liquid flavour and the Sipemat 50S are distributed homogeneously. Finally, the magnesium stearate is added to the blender and mixed for 1 minute.

Tablets are prepared with an individual weight of 2800 mg.

### Example 2 (Film-coated Chewing Gums)

Chewing gums prepared as described in Example 1 are loaded in a coating pan and sprayed on with a suspension composed by:

| | | |
|---|---|---|
| Hypromellose | % | 46,0 |
| Macrogol 6000 | % | 16,0 |
| Titanium dioxide | % | 15,0 |
| Talc | % | 15,0 |
| Saccharin sodium | % | 6,0 |
| Mint Flavour | % | 2,0 |

The coating suspension is applied by means of an automatic spray system until a weight gain of about 200 mg/tablet is reached.

### Example 3 (Chewing Gums)

| | | | |
|---|---|---|---|
| SAMe SD4 | | mg | 192,1 |
| Folic acid | | mg | 0,8 |
| Vitamin B₁₂ | | mg | 1,0 |
| MedGumBase | (Gum Base) | mg | 1650,0 |
| Maltisorb | (Roquette) | mg | 24,1 |
| Sodium bicarbonate | | mg | 62,0 |
| Lemon flavour powder | (Givaudan) | mg | 18,0 |
| Compritol 888 | (Gattefossé) | mg | 20,0 |
| Talc | | mg | 20,0 |
| Magnesium stearate | | mg | 12,0 |
| Talin | | mg | 0,01 |

The gum base (MedGumBase) is loaded in a suitable blender and the lumps, if present, are broken. In the same blender, SAMe SD4, sodium bicarbonate, talc, mint flavour powder, and Talin are incorporated. Following, a pre-mixture of folic acid and vitamin B₁₂ carefully distributed into the Maltisorb is added and mixed. Finally, the magnesium stearate and

Compritol 888 are added to the blender and mixed for 1 minute.

Tablets are prepared with an individual weight of 2000 mg.

### Example 4 (Chewing Gums)

| Core: | | | |
|---|---|---|---|
| SAMe Tosylate | | mg | 384,2 |
| Pharmagum M | (SPI Pharma) | mg | 2018,3 |
| L-Arginine | (Kyowa) | mg | 192,0 |
| Mint flavour powder | (Firmenich) | mg | 20,0 |
| Mint flavour liquid | (Firmenich) | mg | 15,0 |
| Magnesium stearate | | mg | 43,0 |
| Sucralose | (Tate & Lyle) | mg | 7,5 |
| Sipemat 50S | (Evonik Degussa) | mg | 20,0 |

| Coating: | | | |
|---|---|---|---|
| 5-HTP | (5-Hydroxy Tryptophan) | mg | 50,0 |
| Vitamin B₆ | (Pyridoxine Hydrochloride) | mg | 4,0 |
| Vitamin B₁₂ | (Cyanocobalamin) | mg | 0,5 |
| Folic acid | | mg | 0,4 |
| Xylitol | (Xylisorb^{®} - Roquette) | mg | 236,1 |
| Microcrystalline cellulose | | mg | 200,0 |
| Mint Flavour powder | | mg | 4,0 |
| Magnesium stearate | | mg | 5,0 |

The gum base (Pharmagum M) is loaded in a suitable blender and mixed slowly while the mint flavour liquid is added. At the end of this operation Sipemat 50S is added and mixed for about 5 minutes. SAMe tosylate, L-arginine, mint flavour powder, and sucralose are incorporated into the flavoured gum base and blended for about 15 minutes. Finally, magnesium stearate is added and mixed for about 5 minutes. Tablets are prepared with an individual weight of 2700 mg.

The compression coating mixture is prepared by mixing all the components, exception for magnesium stearate, in a suitable blender. Finally, magnesium stearate is added and mixed for about 1 minute. The resulting mixture is then compression coated around the gum cores using a suitable tabletting machine (e.g., Manesty DryCota).

### Example 4 (Orodispersible tablets)

| | | | |
|---|---|---|---|
| SAMe Tosylate | | mg | 194,5 |
| Ludiflash | (BASF) | mg | 380,5 |
| L-Arginine | (Kyowa) | mg | 195,0 |
| Aerosil 200 | (Degussa) | mg | 10,0 |
| Mint Flavour | (Givaudan) | mg | 5,0 |
| Aspartame | (Ajinomoto) | mg | 6,0 |
| Talin | (Overseal) | mg | 0,01 |
| Sodium stearyl fumarate | (JRS Pharma) | mg | 9,0 |
| Tablet weight: | | mg | 800 |

Talin, aspartame, mint flavour and Aerosil 200 are sieved and pre-mixed. SAMe tosylate, Ludiflash, and L-arginine are loaded in a suitable blender and mixed. Finally, sodium stearyl fumarate is added in the same blender and mixed for about 3 minutes. The resulting mixture is compressed in a tabletting machine.

### Example 5 (Orodispersible tablets)

| | | | |
|---|---|---|---|
| SAMe SD4 | | mg | 192,1 |
| F-Melt | (Fuji Chem.Ind.) | mg | 847,5 |
| L-Lisine | (Kyowa) | mg | 80,0 |
| Aerosil 200 | (Degussa) | mg | 10,0 |
| Lemon Flavour | (Firmenich) | mg | 25,0 |
| Noveon CA-2 | (Lubrizol) | mg | 30,0 |
| Aspartame | (Ajinomoto) | mg | 7,0 |
| Magnesium stearate | | mg | 8,4 |
| Tablet weight: | | mg | 1200 |

All components, except magnesium stearate, were loaded into a suitable blender and mixed. Then, magnesium stearate is added and mixed for about 1 minute. The resulting mixture is compressed in a tabletting machine.

### Example 6 (Chewable Tablets)

| | | | |
|---|---|---|---|
| SAMe Tosylate | | mg | 384,2 |
| Sorbitol | (Roquette) | mg | 968,8 |
| Mannitol | (Roquette) | mg | 200,0 |
| L-Arginine | (Kyowa) | mg | 192,0 |
| Aerosil 200 | (Degussa) | mg | 10,0 |
| Mint Flavour | (Firmenich) | mg | 20,0 |
| Acesulfame K | | mg | 7,0 |
| Sodium stearyl fumarate | (JRS Pharma) | mg | 18,0 |
| Talin | (Overseal) | mg | 0,01 |
| Tablet weight: | | mg | 1800 |

Talin, acesulfame K, and Aerosil 200, are sieved and premixed. SAMe tosylate, sorbitol, mannitol, L-arginine and mint flavour are loaded in a suitable blender and mixed. Finally, sodium stearyl fumarate is added in the same blender and mixed for about 3 minutes. The resulting mixture is compressed in a tabletting machine.

**Table 1**

| Batch No. | Time = 0 | Time = 24 h |
|---|---|---|
| P026/1 | 0,79 | 0,91 |
| P026/2 | 0,92 | 0,99 |
| P027/1 | 1,18 | 1,27 |
| P027/2 | 1,08 | 1,32 |

### Method:

Chewing gums were stored at 40°C and 75% r.h. on a Petri dishes for 24 hours then the KF moisture was checked in comparison with an untreated sample.

Notes:
P026/1 = Chewing gums made with SAMe Tosylate
P026/2 = Chewing gums made with SAMe SD4
P027/1 = Film coated chewing gums made with SAMe Tosylate
P027/2 = Sugarless coated chewing gums made with SAMe Tosylate

## Claims

1. Formulations for systemic buccal delivery comprising as active principle sulpho-adenosyl-L-methionine (SAMe) capable of allowing the adsorption of said active principle through the oral mucosa.

2. Formulations according to claim 1 in the form of: chewing gums, chewable tablets, orodispersible tablets, oromucosal preparations.

3. Formulations according to claims 1 and 2 in which the quantity of active principle is comprised between 50 and 1000 mg, preferably is comprised between 100 and 800 mg, and more preferably is comprised between 200 and 400 mg.

4. Formulations according to claims 1 - 3 comprising also vitamins, aminoacids, mineral salts or oligoelements and pH modifying agents chosen among amino acids, carbonates or bicarbonate, phosphate or citrate salts5.

5. Formulations according to Claim 4 contain a sweetener or a high-intensity sweetener chosen among aspartame, acesulfame, saccharin and its salts, cyclamic acid and its salts, glycyrrhizin, sucralose, thaumatin, and mixtures thereof.

6. Formulations according to claims 2 - 5 **characterised in that** the chewing gum have a sugar-coating, a sugar-less coating ,a film-coating.

7. Formulations according to Claims 2 - 6 wherein the chewing gum comprises two or more layer.

8. Formulations according to Claim 7 wherein one layer is a non-rubber layer.

9. Formulations according to Claim 8 wherein the non-rubber layer comprises vitamins, mineral salts oligoelements.

10. Process for the preparations of formulations wherein the SAMe, as well as the other active ingredients if present, is mixed with the directly compressible gum together with the other appropriate excipients for a time sufficient for reaching the uniformity of distribution and thereafter the mixture is loaded into a tabletting machine and compressed following standard procedures and possibly coated.

11. A method for the treatment of depression comprising the steps of:
providing a chewing gum composition that includes a therapeutically effective amount of SAMe in the chewing gum composition; and
chewing the chewing gum composition to cause the SAMe to be released from the chewing gum composition into the oral cavity of the individual.
